# EUROPEAN PATENT SPECIFICATION

(11) **EP 0 680 277 B1**
(45) Date of publication and mention of the grant of the patent: **16.06.1999**
(21) Application number: 94907339.9
(22) Date of filing: 25.01.1994
(51) Int. Cl.: A61B 6/00, A61B 6/12, A61M 25/01

(54) **CATHETER INCLUDING AN X-RAY SENSITIVE OPTICAL-SENSOR LOCATING DEVICE**
KATHETER MIT RÖNTGEN-EMPFINDLICHER, OPTISCHER POSITIONIER-VORRICHTUNG
CATHETER COMPRENANT UN DISPOSITIF DE LOCALISATION D'UN CAPTEUR OPTIQUE SENSIBLE AUX RAYONS X

(30) Priority: 25.01.1993 US 8455
(43) Date of publication of application: 08.11.1995
(73) Proprietor: CARDIAC MARINERS INCORPORATED, Los Gatos, CA 95032 (US)
(72) Inventor: MOORMAN, Jack, Wilson, Los Gatos, CA 95032 (US); WILENT, John William, deceased (US)
(74) Representative: Viering, Jentschura & Partner
(86) International application number: US9400957
(87) International publication number: WO9416623

(56) References cited:
- EP-A- 0 255 369
- DE-A- 3 833 365
- US-A- 3 992 631
- US-A- 4 945 894
- US-A- 5 166 073

## Description

The present invention relates to an x-ray sensor and an x-ray sensing catheter provided with said, x-ray sensor.

The present invention generally relates to identifying the unique location of a marker transported in a maneuverable positioner by use of an x-ray system. More particularly, the present invention relates to devices incorporating such a marker such as a medical catheter which includes an x-ray sensitive optical sensor locating device, useful in diagnostic or therapeutic x-ray procedures in conjunction with scanning beam x-ray apparatus.

An endoscope with an x-ray non-transmitting material either as a filler or coating allows the position of the distal end of the endoscope to be determined by use of x-ray imaging is known from US-A-4,945,894.

From US-A-5,166,073 an optical sensor useful for the detection of ionizing radiation emitted from an analyte is known. The optical signal generated by means of the ionizing radiation is transmitted through an optical fiber.

In numerous fields of application, including medical and industrial, it is desirable to discern the location of internal features of an object. In some of these applications, insertion of an x-ray sensitive device is feasible. Industrial fields are variously called x-ray inspection, x-ray analysis, failure analysis, and in-situ testing. In the medical field, examples include, but are not limited to, interventional cardiology, interventional etectrophysiology,interventional radiology and interventional neurology.

For example, until 1982 the field of etectrophysiology was a small subspecially of cardiology. Physicians in this field performed studies of the electrical function of the heart. Based on their studies, referring physicians might perform open chest surgery, prescribe antiarrhythmic drugs, or take no therapeutic action. These studies were carried out using intracardiac electrodes mounted on catheters and with surface electrodes. The intracardiac electrodes could not only measure cardiac action potentials but could also provide stimulation to pace the heartbeat.

Since 1982 there has been increasing use of catheter ablation to cure certain types of arrhythmia. In these types of arrhythmia, such as Wolff-Parkinson-White syndrome, the conductive congenital muscle fibers can be made nonconductive by heating them locally to a sufficient temperature to cause scar tissue to form. Most of these ablations are done with radio-frequency energy but the emitting electrode must be placed within one to three millimeters of the fiber location and it must stay in intimate contact for a number of heartbeats and respiratory cycles.

In 1989 a study of certain antiarrhythmic drugs was prematurely terminated due to a higher mortality for patients taking the drugs than the controls. These findings have caused increased conservatism prescribing drugs and has increased the interest and use of catheter ablation. With the advent of the automatic implantable Cardioverter Defibrillator (AICD), electrophysiologists have taken a lead role in performing the studies which must be performed prior to their implantation.

Although the treatment of arrhythmia through catheter ablation has some advantages, there are also some problems. The advantages are a very high success rate, the procedure is minimally invasive, can be performed in a few hours in a procedure room, and is considerably less expensive than open chest surgery or a lifetime of drugs. The major disadvantages are that the length of the procedure is both uncertain and typically long. This leads to higher costs, inability to schedule physicians and facilities, fatigue for both patient and staff, and high radiation dosages.

Attempts to solve these problems have focused mainly on providing more steerable catheters to reduce the time to find the precise location of the ablation site and to position the catheter for remaining in contact with the substrate during the ablation time, which is typically five to ninety seconds for each "buzz". Having more steerable catheters has not yet reduced the time or uncertainty of time because the location of the catheter can only be generally determined by looking at an x-ray projected on a monitor and by analyzing the electrocardiogram. Both of these actions must be done in real time in order to know whether to move the catheter and in which direction to move it. The actual direction of movement is uncertain due to the nature of an x-ray image of soft tissue and blood, the poor control and feedback of the catheter, the movement of the heart, and the difficulty of determining direction from the electrocardiogram analysis.

In the U.S., there are currently 300,000 to 500,000 people who die each year due to arrhythmia that is a result of a myocardial infarction. There is no cure for the problem. However, it is believed that if the slow conduction zone around the infarct could be electrically mapped and selectively ablated, that the cure would be obtained. Tests on animals and some humans have demonstrated the possibility of such a procedure but the success rate has been low. The reason for the low success is thought to be the need to map the entire area of the infarct and slow conduction zone and then to be able to ablate multiple sites without depending on acquiring a characteristic electrogram once the ablation has begun. There are current investigations attempting to solve the problem utilizing a network of nodes but this has the problem of extracting the nodal network array from inside the heart without damaging the heart.

It is therefore one object of the present invention to provide a catheter whose position may be accurately determined during a medical diagnostic or therapeutic procedure.

This object is achieved by an x-ray sensor according to claim 1 and an x-ray sensing catheter according to claim 5 provided with an x-ray sensor.

In other words, one embodiment of the present invention relates to a marker transported in a maneuverable positioner which allows identification of its unique location by use of an x-ray system. In its most general sense, the present invention comprises an x-ray sensitive marker material disposed in a body and includes means for transmitting an indication of the presence of x-ray radiation outside of the body in which it is disposed. As presently preferred, the marker material is a scintillating crystal or phosphor material optically coupled to one end of an optical fiber which extends beyond the boundaries of the body. The other end of the optical fiber may be optically coupled to a photodetector. Alternatively, the marker may comprise a transducer for converting x-ray radiation to an electrical signal and may be electrically coupled to a wire or wires which are used to transmit the electrical signal beyond the boundaries of the body in which it is contained.

According to one embodiment of the invention useful in medical applications, a catheter comprises an elongated body having a distal end adapted to be inserted into a bodily cavity, vessel, tract, or the like and a proximate end available to a person performing a medical procedure. The catheter includes at least one lumen running therethrough. An optical fiber is disposed in the at least one lumen and extends from the distal end to the proximate thereof. An x-ray sensitive phosphor material is disposed at the tip of the end of the optical fiber positioned at the distal end of the catheter and the end of the optical fiber at the proximate end of the catheter is coupled to a photodetector.

The principles of the present invention may be employed in any application, medical or industrial, where location of internal features of an object is desired and insertion of an x-ray sensitive device is feasible. Industrial fields are variously called x-ray inspection, x-ray analysis, failure analysis, and in-situ testing.

### BRIEF DESCRIPTION OF THE DRAWINGS

FIG 1 is a diagram of a catheter according to a presently preferred embodiment of the present invention.
FIG. 2 is a cross-sectional view of the catheter of FIG. 1.
FIG. 3 is a detailed view of the distal end of the catheter of FIGS. 1 and 2.
FIG. 4a is a diagram of an electrophysiology catheter employing the present invention.
FIG. 4b is a diagram of a balloon angioplasty catheter employing the present invention.
FIG. 4c is a diagram of an atherocatheter employing the present invention.
FIG. 4d is a diagram showing how the x-ray markers of the present invention may be used to determine the rotational orientation of the catheter in which they are employed
FIG. 5 is a block diagram illustrating an application of the present invention with a digital scanning beam x-ray system.
FIG. 6 is a block diagram similar to that of FIG. 5, illustrating an application of the present invention with two digital scanning beam x-ray systems for locating the marker in three dimensions.

### MODES FOR CARRYING OUT THE INVENTION

Those of ordinary skill in the art will realize that the following description of the present invention is illustrative only and not in any way limiting. Other embodiments of the invention will readily suggest themselves to such skilled persons. While the preferred embodiment of the invention contained herein is disclosed in the context of medical catheters specifically as applied to catheter ablation of cardiac arrhythmias, those of ordinary skill in the art will readily recognize that the present invention is generally and broadly applicable to x-ray markers disposed in positionable bodies.

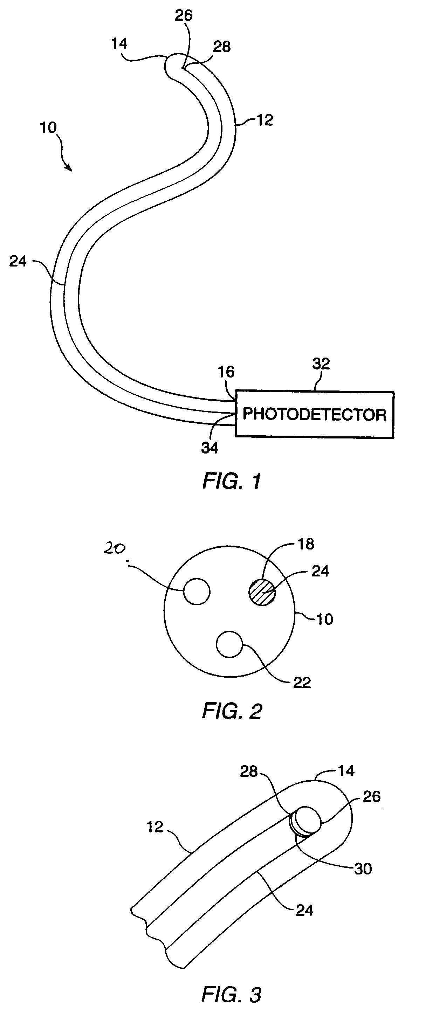

Referring first to FIGS. 1 and 2, diagrams are presented of a catheter 10 according to a presently preferred embodiment of the invention. As shown in FIG. 1 and as will be appreciated by those of ordinary skill in the art, catheter 10 is an elongated body which may be formed from a variety of materials, such as plastic and steel. Catheter 10 comprises an elongated body 12 having a distal end 14 adapted to be inserted into a bodily cavity, vessel, tract, or the like and a proximate end 16 available to a person performing a medical procedure.

Referring particularly to FIG. 2, catheter 10 is shown to include a plurality of lumens 18, 20, and 22. While the catheter 10 is shown including three lumens 18, 20, and 22, those of ordinary skill in the art will readily recognize that this embodiment is merely illustrative to point out the features of the invention and that the number of lumens provided in any actual catheter fabricated according to the present invention and the uses therefore will depend solely on the end use to which catheter 10 will be put. Such a catheter may be steerable to be positioned by manipulation as is known in the art.

Referring again to FIG. 1, an optical fiber 24 is disposed in lumen 18 of catheter 10. For small diameter catheters, such as ones used in cardiac applications, optical fiber 24 may have a diameter of approximately 0.025-0.051 cm (0.010-0.020 inch). Those of ordinary skill in the art will of course recognize that optical fiber 24 may have other diameters without departing from the scope of the present invention. An x-ray marker 26, preferably comprising a sensitive phosphor material or crystal, is optically coupled to a distal end 28 of the optical fiber 24. X-ray marker material 26 may comprise a material such as terbium-doped gadolinium oxysulfate (Gd₂O₂S₂:Tb), available from USR Optronix, Inc. of Hackettstown, New Jersey. Those of ordinary skill in the art will recognize that other scintillating phosphors, scintillating crystals, and other materials or devices which are responsive in the x-ray spectrum may also be used in the present invention and are to be considered as x-ray marker materials within the meaning of that phrase as used herein.

Referring to FIG. 3, in an actual embodiment fabricated according to the teachings of the present invention, terbium doped gadolinium oxysulfate phosphor material in a finely ground powder form was both physically mounted and optically coupled to the distal 28 end of optical fiber 24 using an epoxy 30, such as QUIKSTIK, available from GC Electronics of Rockford, Illinois. The epoxy 30 was used to adhere the x-ray marker material 26 to the optical fiber 24 by placing a small amount of the epoxy 30 at the distal end of optical fiber 24 and then dipping the distal end 28 of optical fiber 24 into a finely divided phosphor powder. Similar techniques may be employed to affix a scintillating crystal to the distal end 28 of optical fiber 24. Those of ordinary skill in the art will recognize that the epoxy 30 must be compatible with the phosphor material and must be optically clear at the phosphor emission wavelength. In addition, such skilled persons will recognize that other methods for optically coupling the marker material to the optical fiber, such as employment of a lens, are equivalent and fall within the scope of the present invention.

A photodetector 32 is coupled to the proximate end 34 of optical fiber 24. In an actual embodiment fabricated according to the present invention, the proximate end 34 of optical fiber 24 was coupled to the light-sensitive window of an RCA XT-2020 photomultiplier tube using a high viscosity clear silicone oil, such as available from Dow Corning Corporation of Midland, Michigan.

As will be appreciated by those of ordinary skill in the art, an electrical signal will be generated in photodetector 32 when the catheter 10 of the present invention is exposed to x-ray radiation. Catheter 10 of the present invention is therefore highly useful when employed in applications with digital scanning beam x-ray apparatus, since the x-ray emission is in the form of discrete pixels, enabling location of the catheter's position with a high degree of accuracy.

Those of ordinary skill in the art will appreciate that, although the catheter 10 of FIGS. 1-3 is shown employing a single optically-coupled x-ray marker 26, catheters having a plurality of such optically coupled x-ray markers may be fabricated according to the principles of the present invention. The number of such markers employed in any actual embodiment of the catheter of the present invention will depend solely on the application for which the particular catheter is designed. Specific illustrative examples are disclosed herein.

As those of ordinary skill in the art will also recognize, the present invention may be employed in a wide variety of both steerable and non-steerable catheters for various applications. Without limitation, such catheters and applications will include multi-electrode catheters employed in electrophysiology, and catheters for ablation of cardiac arrhythmia, coronary atherocatheters, etc. The present invention will also be usefully employed, for example, in angioplasty balloon catheters, and in laparoscopy equipment. Such catheters now employ bands of radio-opaque material such as metal, so that they may be located by x-ray techniques during performance of angioplasty procedures. Those of ordinary skill in the art will doubtless be able to envision other applications for the present invention.

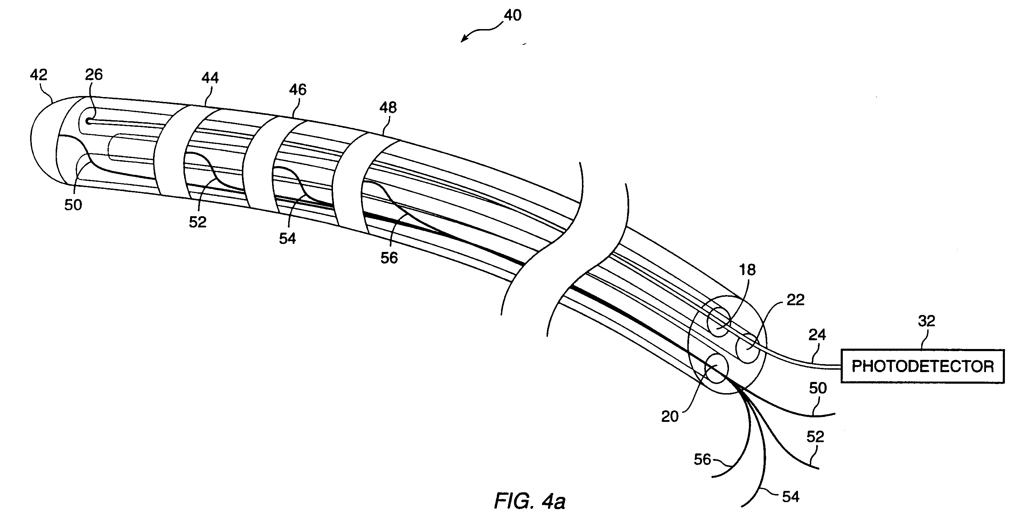

Referring now to FIGS. 4a-4c, certain exemplary catheters employing the present invention will be disclosed. Referring first to FIG. 4a, a multi-electrode electrophysiology catheter 40 for use in measuring intracardiac action potentials incorporates the present invention and is shown diagrammatically. An example of such a Commercial electrophysiology catheter of this type is Steerocath Model No. 2037 available from EP Technologies, Inc. of Mountain View, California. For convenience, features of the catheter 40 which are the same as features of catheter 10 of FIGS. 1-3 are identified by the same reference numerals. Thus, catheter 40 is shown to include a plurality of lumens 18, 20, and 22. An optical fiber 24 is disposed in lumen 18 of catheter 10. An x-ray marker material 26 is affixed to a distal end 28 of the optical fiber 24.

A plurality of conductive electrodes 42, 44, 46, and 48 are disposed in spaced-apart relationship near the distal end 14 of catheter 40. Electrodes 42, 44, 46, and 48 are individually electrically coupled to conductors 50, 52, 54, and 56, respectively. Conductors 50, 52, 54, and 56 are disposed in lumen 20 and emerge from its orifice at the proximate end 16 of catheter 40 and may be suitably connected to an electrophysiology recording and analysis system as is known in the art. As shown in FIG. 4a, a photodetector 32 is coupled to the proximate end 34 of optical fiber 24.

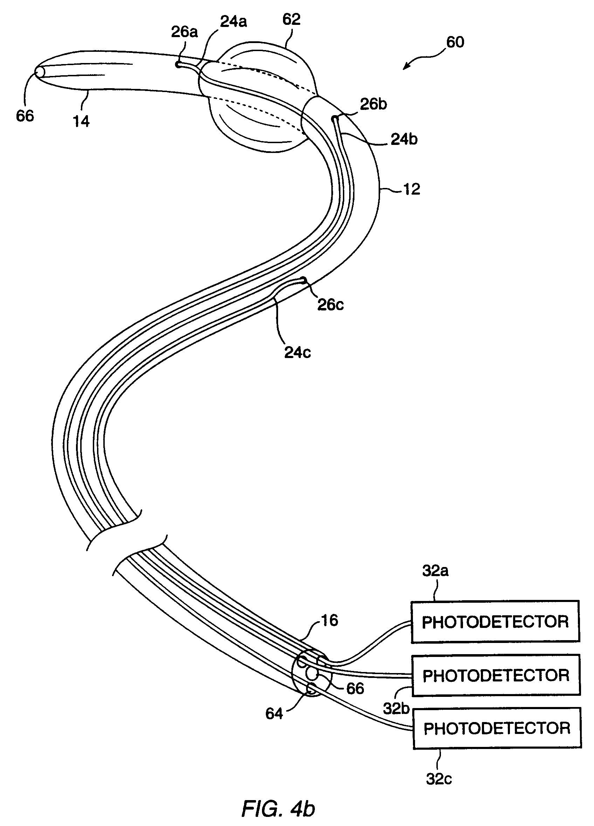

Referring now to FIG. 4b, a diagram of a balloon angioplasty catheter 60 employing the present invention is presented. For convenience, features of the angioplasty catheter 60 which are the same as features of catheter 10 of FIGS. 1-3 are identified by the same reference numerals. Thus, angioplasty catheter 60 comprises an elongated body 12 having a distal end 14 adapted to be inserted into a bodily cavity, vessel, tract, or the like and a proximate end 16 available to a person performing a medical procedure.

As will be recognized by persons of ordinary skill in the art, angioplasty catheter 60 includes an inflatable balloon 62 disposed near its distal end 14 which is used as is known in the art. Balloon 62 may be inflated from a lumen 64 with which it communicates. Angioplasty catheter 60 includes a central guidewire lumen 66 through which a guidewire is threaded as is known in the art.

Angioplasty catheter 60 according to the present invention has advantages over prior art angioplasty catheters because it incorporates a plurality of x-ray markers as taught by the present invention. The embodiment illustrated in FIG. 4b shows three such x-ray markers 26a, 26b, and 26c, but those of ordinary skill in the art will recognize that an arbitrary number of markers could be employed if needed.

In the particular example of FIG. 4b, x-ray marker 26a is disposed near the distal side of the balloon and x-ray marker 26b is disposed near the proximate side of the balloon. In addition, x-ray marker 26c is disposed further down the body 12 of the catheter towards its proximate end 16. X-ray markers 26a, 26b, and 26c are optically coupled to first ends of optical fibers 24a, 24b, and 24c, respectively. Optical fibers 24a, 24b, and 24c are shown disposed in individual lumens 18a, 18b, and 18c, although those of ordinary skill in the art will recognize that they might all be disposed in a single lumen if design considerations warranted such placement.

Optical fibers 24a, 24b, and 24c are connected at their second ends to photodetectors 32a, 32b, and 32c. Those of ordinary skill in the art will recognize that the present invention represents a significant advantage over prior art catheters in which x-ray opaque bands are physically attached to selected positions of the catheter. The catheter of the present invention provides the advantage that the positions of the individual x-ray markers (and thus the position of balloon 62) may be uniquely and accurately determined due to their individual coupling to photodetectors 32a, 32b, and 32c. The present invention is easily interfaced to the widely used digital computer video equipment employed with modern x-ray systems so that, when used in conjunction with a scanning x-ray system, the positions of the x-ray markers may be indicated, for example, by blinking cursors, etc., avoiding the ambiguity which is characteristic of interpretation of x-ray displays to locate prior-art catheters.

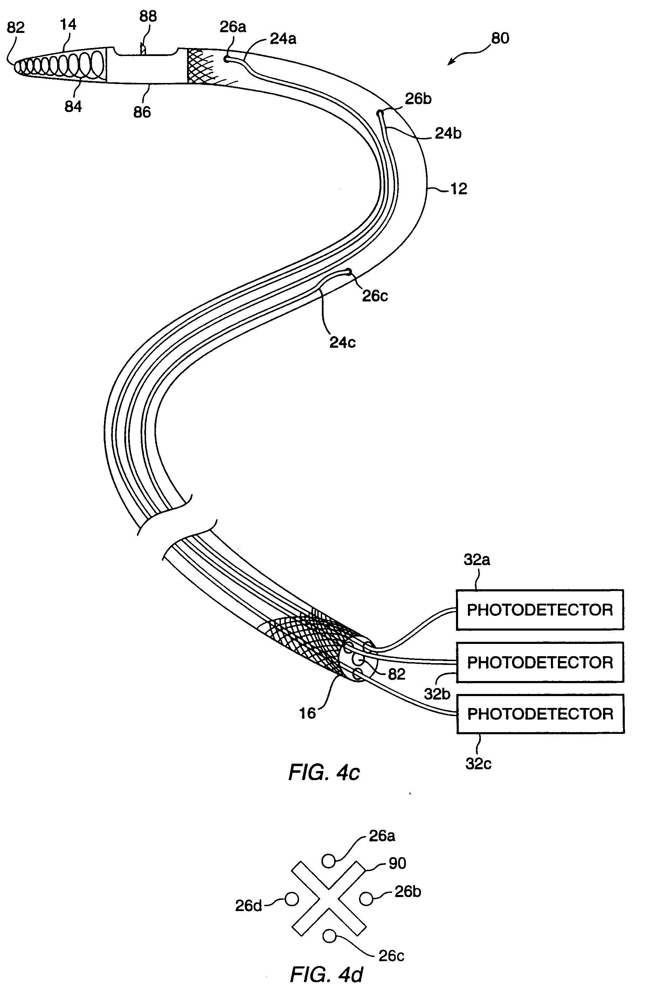

Referring now to FIG. 4c, a diagram of an atherocatheter employing the present invention is presented. For convenience, features of the catheter 40 which are the same as features of catheter 10 of FIGS. 1-3 are identified by the same reference numerals. Thus, atherocatheter 80 comprises an elongated body 12 having a distal end 14 adapted to be inserted into a bodily cavity, vessel, tract, or the like and a proximate end 16 available to a person performing a medical procedure.

A central guidewire lumen 82 runs through the body of atherocatheter 80 from distal end 14 to proximate end 16 as is known in the art. A tapered coil spring 84 is typically internally disposed at the distal end 14 of atherocatheter 80 and functions to provide a stiff tapered end for the catheter in order to stretch the vessel in which it is placed. A rotating cutting section 86 comprises a cylindrical member axially disposed in the catheter body behind the tapered distal end 14. A cutting blade 88 is slidably mounted in rotating cutting section 86. Rotating cutting section 86 is coupled to a shaft 90 which runs through a lumen 92 and may be coupled to a motor for rotation. These elements and their relationship to one another are well known in the prior art.

Atherocatheter 80 according to the present invention has advantages over prior art atherocatheters because it incorporates a plurality of x-ray markers as taught by the present invention. The embodiment illustrated in FIG. 4c shows three such x-ray markers 26a, 26b, and 26c, but those of ordinary skill in the art will recognize that an arbitrary number of markers could be employed if needed.

In the particular example of the atherocatheter of FIG. 4c, x-ray marker 26a is disposed near the distal side of the rotating cutting section 86 and x-ray marker 26b is disposed near the proximate side of the rotating cutting section 86. In addition, x-ray marker 26c is disposed further down the body 12 of the catheter towards its proximate end 16. X-ray markers 26a, 26b, and 26c are optically coupled to first ends of optical fibers 24a, 24b, and 24c, respectively. Optical fibers 24a, 24b, and 24c are shown disposed in individual lumens although those of ordinary skill in the art will recognize that they might all be disposed in a single lumen if design considerations warranted such placement.

Optical fibers 24a, 24b, and 24c are connected at their second ends to photodetectors 32a, 32b, and 32c. Those of ordinary skill in the art will recognize that the present invention represents a significant advantage over prior art catheters in which x-ray opaque bands are physically attached to selected positions of the catheter. The catheter of the present invention provides the advantage that the positions of the individual x-ray markers (and thus of rotating cutting section 86) may be uniquely and accurately determined due to their individual coupling to photodetectors 32a, 32b, and 32c. As in the embodiment of FIG. 4b, the positions of the x-ray markers in atherocatheter 80 of FIG. 4c may be indicated, for example, by blinking cursors, etc., avoiding the ambiguity which is characteristic of interpretation of x-ray displays to locate prior-art catheters.

In the case of the atherocatheter, it is important to know which way the opening for the cutting blade is facing. This can be readily determined according to the present invention by partially shielding the x-ray marker material 26, for example, on three sides, leaving only approximately 90 degrees of rotation open to radiation. If more than one marker is shielded and the openings are oriented in different directions, an accurate assessment of catheter rotational orientation may be made.

This aspect of the present invention is illustrated in FIG. 4d, which is a top view of a plurality of x-ray markers according to the present invention. Although four x-ray markers are depicted in FIG. 4d, those of ordinary skill in the art will recognize that other numbers may be employed to affect the resolution with which the rotational position of the catheter may be determined.

Referring now to FIG. 4d, x-ray markers 26a, 26b, 26c, and 26d are disposed in a shielding member 90, which is illustrated in cross section as generally cross shaped. Shielding member 90 is fabricated from a material which will block x-ray radiation having intensities encountered in the environments within which the catheter employing it will be used. In the typical medical x-ray environment, shield member 90 may comprise, for example, tungsten having a thickness of about 0.025 cm (0.010 in.). It will be possible to utilize other materials and thicknesses because of the ability to compare the outputs of different photodetectors.

Each of x-ray markers 26a, 26b, 26c, and 26d is separately connected to an optical fiber and a photodetector (not shown). In the configuration shown, each of the x-ray markers will be able to determine the rotational position of the catheter in which they are disposed to about a 90° resolution.

Those of ordinary skill in the art will recognize that the top view comprising FIG. 4 does not indicate whether x-ray markers 26a, 26b, 26c, and 26d are disposed at the same point along the length of the catheter in which they are placed. This does not matter and will be a matter of design choice, subject of course to the inaccuracy which would result from twisting of the catheter if they are disposed at different positions along the length of the catheter.

The particular applications and examples disclosed herein are merely illustrative and those of ordinary skill in the art will be readily able to envision other applications for which the present invention is suited. It is intended that all such applications fall within the scope of the present invention.

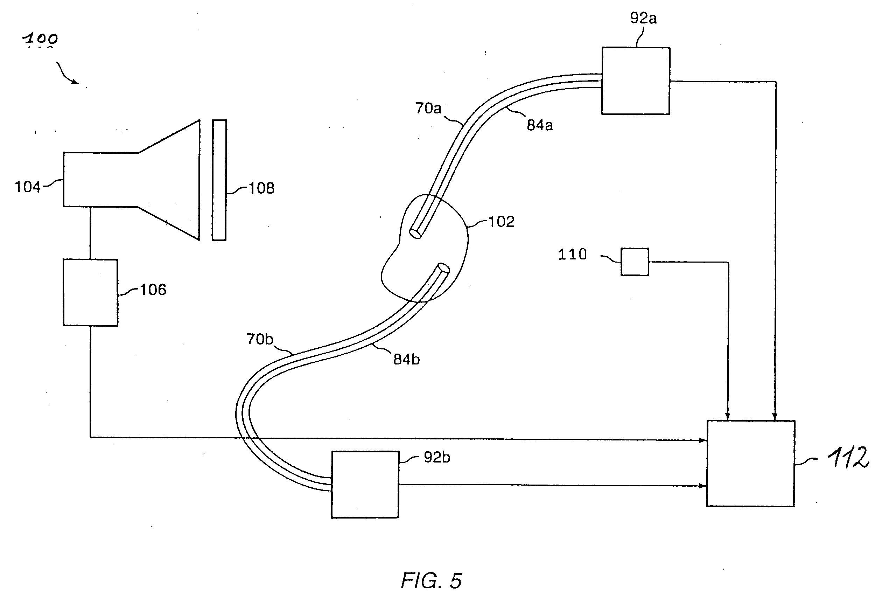

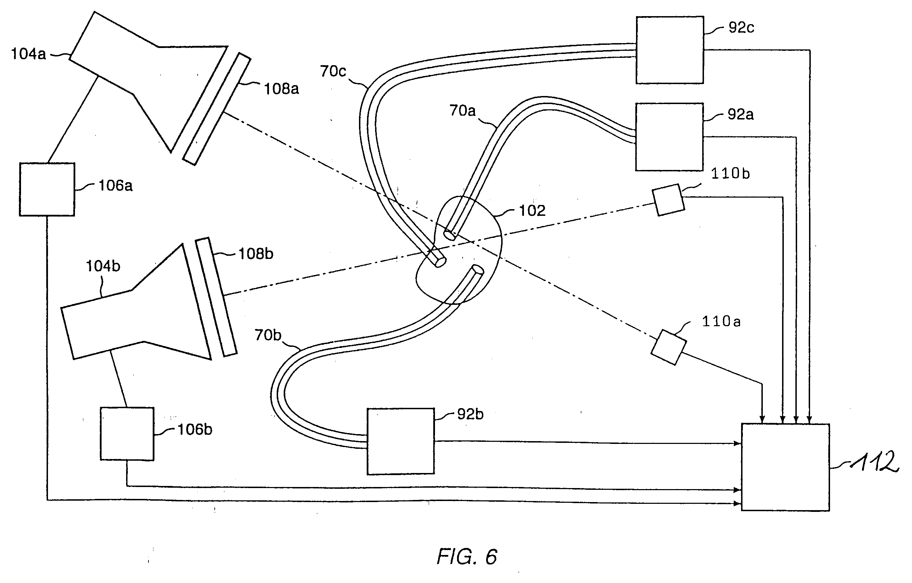

Referring to FIGS. 5 and 6, illustrative uses of catheter 10 of the present invention are shown. Referring first to FIG. 5, a digital scanning beam x-ray system 100 is shown employed with catheters 70a and 70b shown disposed in a volume 102. Those of ordinary skill in the art will readily recognize that volume 102 could be, for example, a patient into which catheters 70a and 70b have been inserted. Photodetectors 92a and 92b are coupled to the proximate ends of the optical fibers inside of catheters 70a and 70b respectively. Two catheters 70a and 70b, and their associated photodetectors 92a and 92b are shown for purposes of illustration, but those of ordinary skill in the art will recognize that any number of catheters and photodetectors may be employed according to the principles of the present invention.

In the digital scanning beam x-ray system 100 shown in FIG. 5, a scanning X-ray source emits radiation in the x-ray spectrum in a conventional manner using a scan pattern in the x-ray source 104 as is known in the art. A digital scan controller 106 controls the position of the beam spot on the face of the x-ray source 104 as is well known in the art. A grid 108 is positioned between the x-ray source 104, and an x-ray detector 110. The grid 108 ensures that x-ray emissions from selected pixel positions of the scanned x-ray tube 104 are directed to a focal point on x-ray detector 110 located behind the volume 102. The x-ray emissions detected by the detector 110 are used to generate a signal which is then sent to an image processor (not shown) for analysis.

As the emissions travel their various paths from the grid 108 to the detector 110, some of the emissions are intercepted by and irradiate the phosphor material disposed on the distal ends of optical fibers 84a and 84b in catheters 70a and 70b, respectively. The phosphor material responds by emitting light, which is directed down the optical fibers 84a and 84b to photodetectors 92a and 92b which convert the light to an electrical signal. Those of ordinary skill in the art will recognize that the signal output from the photodetectors 92a and 92b will be at a maximum when the center of the cone of radiation which is allowed to pass through grid 108 is centered on the phosphor material each catheter.

Control unit 112 is supplied with the x and y addresses from the scan controller 106 and with the output signals from photodetectors 92a and 92b. Control unit 112, which may be a well-known comparator and a well-known coincidence detector, correlates the output signals from photodetectors 32a and 32b with the scan address information from scan controller 106. This information may then be processed and thus may be used to determine the positions of the phosphor material in catheters 70a and 70b, and hence is an accurate indicator of the positions of the ends of the catheters themselves. It will be appreciated that the catheter positions can be located to within one or two pixel positions using the instant invention.

Referring now to FIG. 6, it will be appreciated by those of ordinary skill bin the art that two x-ray sources 104a and 104b, employing independent scan controllers 106a and 106b, grids 108a and 108b, and x-ray detectors 110a and 110b may be used in combination with catheters 70a, 70b, and 70c, photodetectors 92a and 92b, and control unit 112 to accurately locate-the positions of the catheters 70a, 70b, and 70c in three dimensions by employing two of the catheters as reference catheters and a third as a mapping catheter whose position with respect to the other two catheters may be determined.

When used as described herein, the above-described embodiment of the present invention answers the long felt need for anatomical markers during cardiac diagnostic and treatment procedures. For various reasons known to those skilled in the art, the alternative imaging modalities of MRI, CT, and ultrasound are not suitable for these procedures. However, the use of prior-art methods employing x-ray fluoroscopes for imaging has the serious disadvantage of not being able to distinguish anatomical detail inside the heart. The physician uses the shadows generated, his or her intimate knowledge of the anatomy, the characteristic movement of the image and catheters caused by the cardiac cycle and the respiratory cycle, and for fine positioning, the electrocardiogram.

Since typical reference positions for electrocardiograms are the high right atria, the bundle of His, the right ventricle, and the coronary sinus, there is an opportunity to have three points located in the x-ray image at any point in the cardiac cycle. These three points can precisely locate the coordinates of the ablation catheter. The physician can then map an area with the catheter and correlate its position with the electrocardiograms. He can then return to the same spot after leaving it and can measure bounce or other movement, he can also measure internal dimensions of the heart mapping points, determine wall thicknesses, build 3D images from the data and overlay cardiac action potentials. In addition, in a subsequent procedure the same locations may be found from overlaying the maps of anatomy and cardiac potentials.

The initial work performed in this invention was to analyze the images from a biplane x-ray system when it was gated to the cardiac cycle. In some cases this positioning using image analysis is adequate but the precision can be greatly improved if a sensor is included in the catheter which is smaller than the smallest pixel in the output. With conventional image intensifier technology, such a point sensor would not be useful since the entire field of view is irradiated simultaneously. However, in a scanning beam digital x-ray system, the beam is divided such that each pixel is separated in time and therefore the location of the sensor in each individual catheter can be uniquely identified with a pixel. By utilizing a stereo or biplane scanning beam system, the sensor can be located in three dimensions provided that the two beams are synchronized. In cardiac applications, the cardiac cycle must be gated.

The advantage of the above described medical catheter embodiment of the invention is that it permits existing catheters inside the patient to now also function as anatomical markers, significantly reducing the time to map and ablate. Additional advantages are more detailed mapping of the cardiac substrate, correlation of the intercardiac electrodes with anatomical location, display in three dimensions if desired of the intercardiac electrodes on an image of the heart, and comparison of electrograms from studies done at different times by overlaying.

## Claims

1. An x-ray sensor for determining the position of an associated object in a field of view covered by a scanning x-ray beam characterized in that it comprises
an x-ray marker (26) optically coupled to an optical fiber (24) said x-ray marker generating an optical signal in response to x-ray radiation, and
x-ray shielding means (90) for selectively allowing x-ray radiation to reach said x-ray marker (26) only from selected directions.

2. An x-ray sensor as in claim 1, further comprising a photodetector (32) coupled to said optical fiber and responsive to said optical signals.

3. An x-ray sensing catheter as in claim 1 or 2, wherein said x-ray shielding means (90) partially envelops said x-ray marker (26).

4. An x-ray sensing catheter as in claim 3, wherein said selected directions are defined by a conic section having an apex at said x-ray marker.

5. An x-ray sensing catheter characterized in that it comprises
an x-ray sensor according to claim 1,
an elongated body (12) formed from pliant material, said elongated body (12) having a distal end (14), a proximate end and at least one lumen (18) axially disposed therein; wherein
said optical fiber (24) is disposed in said at least one lumen (18), said optical fiber (24) having a distal end disposed at a selected location with respect to said distal end (14) of said elongated body (12) and having a proximate end associated with said proximate end (16) of said elongated body (12); and
said x-ray marker (26) is affixed to said distal end (14) of said optical fiber (24).

6. An x-ray sensing catheter as in claim 5 further comprising at least one conductive electrode (42) disposed on an outer surface of said elongated body (12) near said distal end (14) thereof; and
a conductor (50) electrically connected to said at least one electrode said conductor running through said at least one lumen (18) of said elongated body (12) and accessible at said proximate end (16) of said elongated body (12) for electrical connection thereto.

7. The catheter as in claims 5 or 6 wherein said elongated body (12) has a plurality of lumens (18, 20, 22) axially disposed therein;
a plurality of said x-ray markers (24a, 24b, 24c), each of said x-ray markers disposed in one of said lumens (18, 20, 22) at selected positions along said elongated body;
a pluralitiy of said optical fibers (24), each of said optical fibers disposed in one of said lumens (18, 20, 22) and having a distal end (14) optically coupled to a different one of said x-ray markers (24) and a proximate end associated with said proximate end of said elongated body (12); wherein
said x-ray shielding means (90) are associated with each of said x-ray markers (24), each of said x-ray shielding means (90) partially enveloping its associated x-ray marker (24).

8. A catheter as in claim 7, wherein said selected directions of each of said x-ray shielding means (90) are spaced equidistantly around a circle perpendicular to an axis parallel to said elongated body (12) so as to provide an indication of the rotational position of the catheter.

9. The catheter as in claim 5 or 6 further comprising a photodetector (32) coupled to said proximate end of said optical fiber (24) and responsive to said optical signals.

10. An x-ray sensing catheter as in one of claims 5 to 9, wherein said x-ray shielding means (90) partially envelops said x-ray marker.

11. An x-ray sensing catheter as in one of claims 5 to 10, wherein said selected directions are defined by a conic section having an apex at said x-ray marker and having an axis perpendicular to said elongated body.

## Patentansprüche

1. Röntgensensor zum Bestimmen der Lage eines zugehörigen Objekts in einem von einem Röntgen-Abtaststrahl erfaßten Bildwinkel, dadurch gekennzeichnet, daß dieser
einen Röntgenmarker (26) aufweist, der mit einem Lichtleiter (24) optisch gekoppelt ist, wobei der Röntgenmarker durch Erzeugen eines optischen Signals auf Röntgenstrahlen anspricht, und
eine Röntgen-Abschirmeinrichtung (90), die Röntgenstrahlen nur aus ausgewählten Richtungen selektiv zu dem Röntgenmarker (26) gelangen läßt.

2. Röntgensensor nach Anspruch 1, wobei dieser ferner einen Fotodetektor (32) aufweist, der mit dem Lichtleiter gekoppelt ist und auf die optischen Signale anspricht.

3. Röntgensensor-Katheter nach Anspruch 1 oder 2, wobei die Röntgenstrahlen-Abschirmeinrichtung (90) den Röntgenmarker (26) teilweise umhüllt.

4. Röntgensensor-Katheter nach Anspruch 3, wobei die ausgewählten Richtungen von einem konischen Abschnitt festgelegt werden, der an dem Röntgenmarker eine Spitze aufweist.

5. Röntgensensor-Katheter, dadurch gekennzeichnet, daß dieser
einen Röntgensensor nach Anspruch 1 aufweist, ferner
einen aus biegsamem Material geformten länglichen Körper (12), wobei der längliche Körper (12) ein distales Ende (14), ein proximales Ende und mindestens ein axial in seinem Inneren angeordnetes Lumen (18) aufweist, wobei
der Lichtleiter (24) in dem wenigstens einen Lumen (18) angeordnet ist und ein distales Ende aufweist, das bezüglich des distalen Endes (14) des länglichen Körpers (12) an einer ausgewählten Stelle angeordnet ist, sowie ein proximales Ende, das dem proximalen Ende (16) des länglichen Körpers (12) zugeordnet ist, und
der Röntgenmarker (26) an dem distalen Ende (14) des Lichtleiters (24) befestigt ist.

6. Röntgensensor-Katheter nach Anspruch 5, wobei dieser ferner wenigstens eine leitfähige Elektrode (42) aufweist, die an einer Außenfläche des länglichen Körpers (12) in der Nähe von dessen distalem Ende (14) angeordnet ist, und
einen Leiter (50), der mit der wenigstens einen Elektrode elektrisch verbunden ist, wobei der Leiter durch das wenigstens eine Lumen (18) des länglichen Körpers (12) verläuft und an dem proximalen Ende (16) des länglichen Körpers (12) für einen elektrischen Anschluß daran zugänglich ist.

7. Katheter nach Anspruch 5 oder 6, wobei der längliche Körper (12) eine Mehrzahl von axial in diesem angeordneten Lumina (18, 20, 22) aufweist, ferner
eine Mehrzahl der Röntgenmarker (24a, 24b, 24c), wobei jeder dieser Röntgenmarker in jeweils einem der Lumina (18, 20, 22) an einer ausgewählten Stelle entlang des länglichen Körpers angeordnet ist,
eine Mehrzahl der Lichtleiter (24), wobei jeder dieser Lichtleiter in jeweils einem der Lumina (18, 20, 22) angeordnet ist und ein distales Ende (14) aufweist, das mit jeweils einem anderen der Röntgenmarker (24) optisch gekoppelt ist, sowie ein proximales Ende, das dem proximalen Ende des länglichen Körpers (12) zugeordnet ist, wobei
die Röntgen-Abschirmeinrichtungen (90) jedem der Röntgenmarker (24) zugeordnet sind, und jede der Röntgen-Abschirmeinrichtungen (90) den ihr zugeordneten Röntgenmarker (24) teilweise umhüllt.

8. Katheter nach Anspruch 7, wobei die ausgewählten Richtungen aller Röntgen-Abschirmeinrichtungen (90) in gleichen Abständen voneinander auf einem Kreis liegen, der rechtwinklig zu einer dem länglichen Körper (12) parallelen Achse liegt, so daß man einen Hinweis auf die Rotationslage des Katheters erhält.

9. Katheter nach Anspruch 5 oder 6, wobei dieser ferner einen Fotodetektor (32) aufweist, der mit dem proximalen Ende des Lichtleiters (24) gekoppelt ist und auf die optischen Signale anspricht.

10. Röntgensensor-Katheter nach einem der Ansprüche 5 bis 9, wobei die Röntgen-Abschirmeinrichtung (90) den Röntgenmarker teilweise umhüllt.

11. Röntgensensor-Katheter nach einem der Ansprüche 5 bis 10, wobei die ausgewählten Richtungen von einem konischen Abschnitt festgelegt werden, der an dem Röntgenmarker eine Spitze aufweist sowie eine rechtwinklig zu dem länglichen Körper liegende Achse hat.

## Revendications

1. Détecteur de rayons X pour déterminer la position d'un objet associé dans un champ d'observation couvert par un faisceau de rayons X de balayage, caractérisé en ce qu'il comprend
un marqueur (26) réagissant aux rayons X couplé optiquement à une fibre optique (24), ledit marqueur réagissant aux rayons X produisant un signal optique en réponse à une irradiation par des rayons X, et
des moyens (90) formant écran contre les rayons X pour permettre sélectivement à un rayonnement formé de rayons X d'atteindre ledit marqueur (26) réagissant aux rayons X uniquement dans des directions sélectionnées.

2. Détecteur de rayons X selon la revendication 1, comprenant en outre un photodétecteur (32) couplé à ladite fibre optique et apte à répondre auxdits signaux optiques.

3. Cathéter sensible au rayonnement X selon la revendication 1 ou 2, dans lequel lesdits moyens (90) formant écran vis-à-vis des rayons X enveloppent partiellement ledit marqueur (26) réagissant aux rayons X.

4. Cathéter de détection de rayons X selon la revendication 3, dans lequel lesdites directions sélectionnées sont définies par une section conique possédant un sommet au niveau dudit marqueur réagissant aux rayons X.

5. Cathéter de détection de rayons X, caractérisé en ce qu'il comporte
un détecteur de rayons X selon la revendication 1,
un corps allongé (12) formé d'un matériau flexible, ledit corps allongé (12) possédant une extrémité distale (14), une extrémité proximale et au moins une lumière (18) disposée axialement dans le corps;
dans lequel
ladite fibre optique (24) est disposée dans ladite au moins une lumière (18), ladite fibre optique (24) possédant une extrémité distale disposée en un emplacement sélectionné par rapport à ladite extrémité distale (14) dudit corps allongé (12) et possédant une extrémité proximale associée à ladite extrémité proximale (16) dudit corps allongé (12); et
ledit marqueur (26) réagissant aux rayons X est fixé à ladite extrémité distale (14) de ladite fibre optique (24).

6. Cathéter de détection des rayons X selon la revendication 5, comprenant en outre au moins une électrode conductrice (42) disposée sur une surface extérieure dudit corps allongé (12) à proximité de ladite extrémité distale (14) de ce dernier; et
un conducteur (50) connecté électriquement à ladite au moins une électrode, le conducteur s'étendant à l'intérieur de ladite au moins une lumière (18) du corps allongé (12) et étant accessible au niveau de ladite extrémité proximale (16) dudit corps allongé (12) pour une connexion électrique à ce dernier.

7. Cathéter selon la revendication 5 ou 6, dans lequel ledit corps allongé (12) possède une pluralité de lumières (18,20,22) disposées axialement;
une pluralité desdits marqueurs (24a, 24b, 24c) réagissant aux rayons X, chacun desdits marqueurs réagissant aux rayons X étant disposé dans l'une desdites lumières (18,20,22) en des positions sélectionnées le long dudit corps allongé;
une pluralité desdites fibres optiques (24), chacune desdites fibres optiques étant disposée dans l'une desdites lumières (18,20,22) possédant une extrémité distale (14) couplée optiquement à l'un différent desdits marqueurs (24) réagissant aux rayons X et une extrémité proximale associée à ladite extrémité proximale dudit corps allongé (12);
dans lequel lesdits moyens (90) formant écran vis-à-vis des rayons X étant associés à chacun desdits marqueurs (24) réagissant aux rayons X, chacun desdits moyens (90) formant écran vis-à-vis des rayons X enveloppant partiellement le marqueur (24) réagissant aux rayons X qui lui est associé.

8. Cathéter selon la revendication 7, dans lequel lesdites directions sélectionnées de chacun desdits moyens (90) formant écran vis-à-vis des rayons X sont espacées de façon équidistante sur un cercle perpendiculaire à un axe parallèle audit corps allongé (12) de manière à fournir une indication de la position en rotation du cathéter.

9. Cathéter selon la revendication 5 ou 6, comprenant en outre un photodétecteur (32) couplé à ladite extrémité proximale de ladite fibre optique (24) et apte à répondre auxdits signaux optiques.

10. Cathéter de détection des rayons X selon l'une des revendications 5 à 9, dans lequel lesdits moyens (90) formant écran pour les rayons X enveloppent partiellement ledit marqueur réagissant aux rayons X.

11. Cathéter de détection des rayons X selon l'une des revendications 5 à 10, dans lequel lesdites directions sélectionnées sont définies par une section conique possédant un sommet au niveau dudit marqueur réagissant aux rayons X comportant un axe perpendiculaire audit corps allongé.
